# EUROPÄISCHE PATENTSCHRIFT

(11) **EP 3 305 761 B1**
(45) Veröffentlichungstag und Bekanntmachung des Hinweises auf die Patenterteilung: **22.04.2020**
(21) Anmeldenummer: 17197925.5
(22) Anmeldetag: 22.08.2014
(51) Int. Cl.: A61K 9/00, A61K 47/20, A61K 9/107, A61K 31/015, A61K 31/198

(54) **VERFAHREN ZUR HERSTELLUNG EINER BETA-CAROTIN ZUBEREITUNG**
PROCESS FOR PRODUCING A BETA-CAROTENE PREPARATION
POCÉDÉ POUR LA PRODCUTION D'UNE PRÉPARATION DE BETA-CAROTÈNE

(30) Priorität: 02.09.2013 AT 6802013
(43) Veröffentlichungstag der Anmeldung: 11.04.2018
(62) Teilanmeldung aus: 14758783.6
(73) Patentinhaber: Inpharsearch AG, 6300 Zug (CH)
(72) Erfinder: FRANTSITS, Werner J., 1130 Wien (AT)
(74) Vertreter: Beer & Partner Patentanwälte KG

(56) Entgegenhaltungen:
- EP-A1- 1 016 404
- DE-A1- 19 609 477
- DE-A1- 19 819 616

## Beschreibung

Die Erfindung betrifft ein Verfahren zum Herstellen einer Zubereitung von Beta-Carotin in Form einer wässerigen Emulsion, die zur parenteralen Verabreichung geeignet ist (DE 196 09 477 A1).

Aus der EP 1 016 404 A1 (AT 408 186 B) ist eine wässerige Zubereitung von Beta-Carotin bekannt, die in der Veterinärmedizin verwendbar ist. Diese wässerige Zubereitung von Beta-Carotin, in der Beta-Carotin als micellare Lösung (Mikroemulsion) vorliegt, enthält anionische oder nichtionische Lösungsvermittler, beispielsweise Polyoxythylen-660-Hydroxystearat und/oder Isopropylmyristat. Die Zubereitung, die beispielsweise 0,1 bis 10 Prozent (w/v) Beta-Carotin enthält, kann weiters Antioxidantien und wenigstens ein Konservierungsmittel enthalten.

Eine in der Veterinärmedizin verwendbare Injektionslösung aus Mehrfachdosisbehältern, die Beta-Carotin enthält, wird von Alvetra und Werfft in Wien unter dem Markennamen "Carofertin" auf den Markt gebracht. Die bekannte Injektionslösung enthält je Milliliter Injektionslösung 10,0 mg Beta-Carotin, 10,0 mg Benzylalkohol, 0,12 mg Ascorbylpalmitat, 0,10 mg Alpha-Tocopherol, Macrogol-15-hydroxystearat (Solutol HS 15), Isopropylmyristat und Wasser für Injektionszwecke.

Bekannt ist, dass Beta-Carotin im Organismus auf zweierlei Weisen wirkt. Einerseits wird es als Provitamin in Vitamin A umgewandelt und entfaltet über diesen metabolischen Schritt seine Wirkung, andererseits greift Beta-Carotin selbst aktiv in den Stoffwechsel ein. Beta-Carotin hat einen stabilisierenden Effekt auf die Corpora lutea der Ovarien, bewirkt die Stimulation des Follikelwachstums und hat einen protektiven und entzündungshemmenden Einfluss auf das Endometrium. Die parenterale Verabreichung von Beta-Carotin beim Schwein eine Woche vor der Deckung erhöht die Wurfgröße. Dieser Effekt ist besonders deutlich bei Alt-Säuen ausgeprägt.

Eine ausreichende Versorgung von Muttertieren mit Beta-Carotin bewirkt außerdem eine deutliche Immunitätssteigerung der Neugeborenen, mit verminderter Anfälligkeit gegen Darminfektionen und somit geringe Verluste in der Aufzuchtphase.

Problematisch bei bekannten Emulsionen von Beta-Carotin ist es, dass das Konservierungssystem nur relativ kurze Zeit, nämlich 12 bis maximal 15 Monate, in geschlossenem Zustand des Behälters stabil ist und nach erstmaligen Anbruch der Injektionslösung bereits innerhalb weniger Tage zusammenbricht, womit der Abbau des Konservierungsmittels und damit der des Wirkstoffes die zugelassenen Grenzen überschreitet.

Insoweit war die Verwendbarkeit der bekannten Beta-Carotin-Emulsionen in der Tiermedizin begrenzt, als eine zu kurze Haltbarkeit zu beachten war, was in der tierärztlichen Praxis zu häufigem Verwerfen von nur wenig angebrauchten Injektionsflaschen und damit zu unnötigen Verlusten führt.

In der EP 1 016 404 A1 und der AT 408 186 B ist erwähnt, dass Emulsionen von Beta-Carotin problematisch sind, da sie eine nur geringe Stabilität gegenüber spontan einsetzender Phasentrennung besitzen. Weiters ist die Beständigkeit von Beta-Carotin gegen Oxidation durch in der Luft enthaltenen Sauerstoff in Emulsionen schlecht.

Die DE 196 09 477 A1 beschreibt wässerige Solubilisate, die zur parenteralen Verabreichung geeignet sind, welche zumindest ein Carotinoid, zumindest ein nicht wasserlösliches Vitamin und einen nichtionogenen Emulgator enthalten. In einer bevorzugten Ausführungsform enthält die Formulierung neben dem Carotinoid (einen) Tocopherol(ester), Ascorbinsäure sowie gegebenenfalls N-Acetylcystein. Als nichtionogener Emulgator wird Polyoxyethylen-12-hydroxystearat genannt. Die aus DE 196 09 477 A1 bekannte Formulierung ist zum sofortigen Verbrauch bestimmt und für das Bereitstellen in einem Behältnis für Mehrfachdosis nicht geeignet.

Die DE 198 19 616 A1 offenbart eine Zusammensetzung zur Behandlung/Prophylaxe entzündlicher Hautkrankheiten, die N-Acetylcystein und zumindest ein weiteres Antioxidationsmittel ausgewählt aus der Gruppe Ascorbinsäure, α-Tocopherol, β-Carotin und/oder Derivate selbiger enthält. Die Zusammensetzung kann parenteral verabreicht werden und ist nur für das Bereitstellen in einem Behältnis für Einmaldosis geeignet.

Gegenstand der DE 197 47 546 A1 ist die Verwendung von systemisch verabreichten, wasserlöslichen Antioxidantien (z. B. Ascorbat, N-Acetylcystein) gemeinsam mit lipidlöslichen Antioxidantien (z. B. Carotinoide, Tocopherol) zur Behandlung entzündlicher Dermatosen. Diese Zubereitung ist nur für das Bereitstellen in einem Behältnis für als Einmaldosis geeignet.

Der Erfindung liegt die Aufgabe zugrunde, ein Verfahren zum Herstellen einer zur parenteralen Verabreichung, vor allem in der Veterinärmedizin, geeigneten wässerigen Zubereitung von Beta-Carotin zur Verfügung zu stellen, wobei die Zubereitung in Mehrdosisbehältnissen bereitgestellt werden kann.

Gelöst wird diese Aufgabe erfindungsgemäß mit einem Verfahren mit den Merkmalen von Anspruch 1.

Der Erfindung liegt die überraschende Erkenntnis zugrunde, dass der Zusatz von Acetylcystein zu Emulsionen, die Beta-Carotin enthalten, einen stark stabilisierenden Einfluss auf das Konservierungssystem ausübt und damit die Stabilität der Emulsion verbessert, wenn die Zubereitung ein Antioxidans enthält.

Acetylcystein (L-α-Acetamido-β-mercaptoproprionsäure) ist ein an sich bekannter Stoff, insbesondere ein Arzneistoff, der als Expektorans bei Atemwegserkrankungen und als Antidot bei Paracetamol-Intoxikation eingesetzt wird.

Acetylcystein wird auch in der Nephrologie, bei Infektionskrankheiten und in der Psychiatrie angewendet. Acetylcystein ist weder als Stabilisator noch als Konservierungsmittel oder Lösungsvermittler bekannt, so dass der oben geschilderte Effekt der Stabilisierung von Beta-Carotin enthaltenden Emulsionen überraschend ist.

Erfindungsgemäß erhältliche Emulsionen von Beta-Carotin, die zur parenteralen Verabreichung geeignet sind, sind in ungeöffneten Behältnissen (z. B. Mehrdosisbehältnissen) Jahre und nach erstem Anbruch mindestens 8 Wochen stabil, was es erlaubt, die Emulsion zur Fertilitätssteigerung, insbesondere bei Rind und Schwein, sowie zum Behandeln von Störungen im Fertilitätstrakt bei Hunden einzusetzen.

Im Rahmen der Erfindung ist insbesondere in Betracht gezogen, dass der Gehalt von Acetylcystein im Bereich von 1 Gew.% bis 8 Gew.%, beispielsweise bei 3 Gew.%, bezogen auf die Emulsion liegt.

Weitere mögliche Bestandteile der erfindungsgemäßen, wässerigen Emulsion von Beta-Carotin sind neben Beta-Carotin insbesondere Ascorbylpalmitat, Alpha-Tocopherol, Benzylalkohol, Lösungsvermittler wie Isopropylmyristat, wenigstens ein nicht-ionischer Lösungsvermittler, wie Macrogol-15-hydroxyolearat (Solutol HS15), und Wasser.

Als zusätzliches Konservierungsmittel kann die erfindungsgemäße Zubereitung Benzylalkohol enthalten.

Eine erfindungsgemäß erhältliche wässerige Emulsion von Beta-Carotin enthält bevorzugt Beta-Carotin je 1000 g Emulsion in Mengen von 5 bis 15 g, insbesondere in einer Menge von 10 g je 1000 g Emulsion.

Als Antioxidans können Ascorbylpalmitat und Alpha-Tocopherol, beispielsweise in Mengen von 0,05 bis 0,50 g, vorzugsweise 0,12g (Ascorbylpalmitat) und 0,05 bis 0,20 g, vorzugsweise 0,10g (Alpha-Tocopherol) je 1000 g Emulsion, eingesetzt werden.

Das als weiterer Lösungsvermittler gegebenenfalls zugesetzte Isopropylmiristat kann in Mengen von 70 bis 90 g, insbesondere 84 g je 1000 g Emulsion enthalten sein.

Das die Emulsion stabilisierende und Beta-Carotin vor Abbau schützende Acetylcystein (Konservierungsmittel) kann in Mengen zwischen 1 und 8 g, insbesondere 3 g, je 1000 g Emulsion vorliegen.

Beim erfindungsgemäßen Verfahren zum Herstellen der erfindungsgemäßen wässerigen Emulsion von Beta-Carotin, wird der Schritt des Zusetzens von Isopropylmyristat zu dem, beispielsweise geschmolzenen und vorzugsweise nicht-ionischen, Lösungsvermittler (z.B. Solutol) bei einer Temperatur zwischen 25° Celsius und 40° Celsius, vorzugsweise bei 30° Celsius, ausgeführt.

Nachstehend werden weiteren Einzelheiten der Erfindung, an Hand bevorzugter Ausführungsbeispiele beschrieben.

### Beispiel 1:

Eine wässerige, erfindungsgemäß erhältliche Emulsion von Beta-Carotin, die in der Veterinärmedizin zur parenteralen Verabreichung geeignet ist, hat die folgende Zusammensetzung: Beta-Carotin 10,0 g, Ascorbylpalmitat 0,12 g, Alpha-Tocopherol 0,10 g, Benzylalkohol 10,0 g, Isopropylmyristat 84,0 g, Acetylcystein 3,0 g, Solutol HS 15 182,0 g, Wasser 711,0 g. Zusammen 1000,0 g.

### Beispiel 2:

Eine wässerige, erfindungsgemäß herstellbare Emulsion von Beta-Carotin, die in der Veterinärmedizin zur parenteralen Verabreichung geeignet ist, hat die folgende Zusammensetzung:
Beta-Carotin 15,0 g, Ascorbylpalmitat 0,18 g, α-Tocopherol 0,22 g, Isopropylmyristat 90,0 g, Acetylcystein 4,5 g, Solutol HS 15 200,0 g, Wasser 690,1 g.
Zusammen 1000,0 g.

### Beispiel 3:

Eine wässerige, erfindungsgemäß herstellbare Emulsion von Beta-Carotin, die in der Veterinärmedizin zur parenteralen Verabreichung geeignet ist, hat die folgende Zusammensetzung: Beta-Carotin 7,5 g, Ascorbylpalmitat 0,15 g, α-Tocopherol 0,10 g, Isopropylmyristat 75,0 g, Acetylcystein 2,0 g, Solutol HS 15 175,0 g, Wasser 740,25 g.
Zusammen 1000,0 g.

### Beispiel 4:

Eine wässerige, erfindungsgemäß herstellbare Emulsion von Beta-Carotin, die in der Veterinärmedizin zur parenteralen Verabreichung geeignet ist, hat die folgende Zusammensetzung:
Beta-Carotin 5,0 g, Ascorbylpalmitat 0,10 g, α-Tocopherol 15,0 g, Isopropylmyristat 80,0 g, Acetylcystein 6,0 g, Solutol HS 15 180,0 g, Wasser 713,9 g.
Zusammen 1000,0 g.

### Beispiel 5:

Die in Beispielen 1 bis 4 beschriebenen, wässerigen Emulsionen von Beta-Carotin können wie folgt hergestellt werden:
Als nicht-ionischer Lösungsvermittler wird Solutol HS 15 (2-Hydroxyethyl-12-hydroxyoktadekanoat, Macrogol-15-hydroxystearat) im Ansatzkessel auf 60° Celsius erwärmt, schmilzt und ist dann dünnflüssig.

Zu dem geschmolzenen Solutol HS 15 wird flüssiges Isopropylmyristat zugegeben. Das Gemisch wird unter mäßigen Rühren auf 130° Celsius erhitzt.

Der so erhaltenen Lösung wird bei erhöhter Temperatur der Lösung und unter mäßigem Rühren Beta-Carotin langsam zugesetzt und so lange weitergerührt, bis eine dunkelrote, klare Lösung vorliegt. Dabei kann unter Stickstoffatmosphäre gearbeitet werden. Während dieser Arbeitsschritte (Zugeben der öligen Mischung von Beta-Carotin und nachfolgendem Rühren) wird eine Temperatur von 90° Celsius eingehalten. Dabei kann unter Stickstoffatmosphäre gearbeitet werden.

In einem gesonderten Behältnis wird bei Raumtemperatur Ascorbylpalmitat und Alpha-Tocopherol in Benzylalkohol gelöst.

Nachdem die so erhaltene Beta-Carotin enthaltende Emulsion langsam auf 50° Celsius abgekühlt worden ist, wird die zuvor erhaltene Lösung von Ascorbylpalmitat, Alpha-Tocopherol in Benzylalkohol unter Rühren in die Emulsion eingebracht. Auch bei diesem Schritt wird bevorzugt unter Stickstoffatmosphäre gearbeitet.

Als nächster Schritt wird Acetylcystein bei einer Temperatur von 30° Celsius in Wasser gelöst.

Sobald die zuvor erhaltene Beta-Carotin enthaltende Emulsion auf 30° Celsius abgekühlt ist, wird die Acetylcysteinlösung, bevorzugt unter Stickstoffatmosphäre, langsam in die Emulsion eingerührt und solange mäßig gerührt, bis eine dunkelrote klare Emulsion vorliegt.

In den nachstehenden Tabellen sind die Ergebnisse von Stabilitätsprüfungen wiedergegeben.

**Tabelle I:**

| Beta-Carotin-Zubereitung gemäß AT 408 186 B1: | | | |
|---|---|---|---|
| | Gehalte nach Produktion | Nach 6 Monaten | Nach 12 Monaten |
| Beta-Carotin (all-trans + cis) | 99,5 - 101 % | 97,5 - 99 % | 96 - 97 % |
| Benzylalkohol | 100 - 101,5 % | 78 - 86 % | 62 - 65 % |
| Ascorbinpalmitat | 99 - 101 % | 69 - 74 % | 58 - 64 % |
| Tocopherol | 99 - 101 % | 98 - 99 % | 95 - 97 % |

**Tabelle II:**

| Erfindungsgemäße Beta-Carotin-Zubereitung: Beispiel 1: | | | |
|---|---|---|---|
| | Gehalte nach Produktion | Nach 6 Monaten | Nach 12 Monaten |
| Beta-Carotin (all-trans+cis) | 99,5 - 101 % | 99,5 - 100,5 % | 99 - 100 % |
| Benzylalkohol | 100 - 101,5 % | 99,5 - 100 % | 98 - 99, 5 % |
| Ascorbinpalmitat | 99 - 101 % | 98 - 100 % | 94 - 97 % |
| Tocopherol | 99 - 101 % | 99 - 100,5 % | 97 - 99, 5 % |

**Tabelle III:**

| Erfindungsgemäße Beta-Carotin-Zubereitung: Beispiel 2: | | | |
|---|---|---|---|
| | Gehalte nach Produktion | Nach 6 Monaten | Nach 12 Monaten |
| Beta-Carotin (all - trans+cis) | 99,7 - 100,6 % | 99, 4 - 99, 9 % | 98, 4 - 99,1 % |
| Benzylalkohol | 100,4 - 100,8 % | 98, 8 - 99,4 % | 97, 9 - 98, 2 % |
| Ascorbinpalmitat | 99,6 - 100,7 % | 97, 6 - 99, 3 % | 93, 4 - 96, 2 % |
| Tocopherol | 100,2 - 101,3 % | 97,8 - 99,4 % | 94,1 - 95,7 % |

**Tabelle VI:**

| Erfindungsgemäße Beta-Carotin-Zubereitung: Beispiel 3: | | | |
|---|---|---|---|
| | Gehalte nach Produktion | Nach 6 Monaten | Nach 12 Monaten |
| Beta-Carotin (all - trans+cis) | 100,6 - 101,3 % | 99,1 - 100,4 % | 98,6 - 99,6 % |
| Benzylalkohol | 100,3 - 100,8 % | 97,1 - 97, 9 % | 95, 6 - 95, 9 % |
| Ascorbinpalmitat | 100,0 - 100,7 % | 95, 5 - 96, 3 % | 93, 0 - 93, 8 % |
| Tocopherol | 99,7 - 100,8 % | 96,9 - 98,0 % | 93,1 - 93, 6 % |

**Tabelle V:**

| Erfindungsgemäße Beta-Carotin-Zubereitung: Beispiel 4: | | | |
|---|---|---|---|
| | Gehalte nach Produktion | Nach 6 Monaten | Nach 12 Monaten |
| Beta-Carotin (all-trans+cis) | 99,9 - 100,4 % | 98,8 - 99,6 % | 98, 8 - 99, 2 % |
| Benzylalkohol | 100,4 - 101,2 % | 96,9 - 97,9 % | 94,7 - 94, 9 % |
| Ascorbinpalmitat | 99,7 - 100,4 % | 97, 8 - 99, 3 % | 93, 3 - 93, 9 % |
| Tocopherol | 100,5 - 101,1 % | 97,5 - 98,1 % | 93, 6 - 94, 2 % |

Die in den Tabellen II bis V nicht genannten Gehalte an Acetylcystein entsprechen den in den Beispielen 1 bis 4 genannten Gehalten.

Bei den Stabilitätsprüfungen waren die Sample-Größen je Versuchsansatz 85 Liter, abgefüllt in 100 ml Durchstichflaschen (ungeöffnet), eingesetzt.

Da in der Tiermedizin Zimmertemperatur als Lagerungsbedingung gewünscht ist, wurden für die Stabilitätsuntersuchung folgende Lagerungsbedingungen eingehalten:
25° Celsius bei 60 % Luftfeuchtigkeit.

Aus den oben, in Tabelle I wiedergegebenen Stabilitätsdaten einer bekannten Beta-Carotin-Zubereitung und in den Tabellen II bis V wiedergegebenen Stabilitätsdaten erfindungsgemäßer Beta-Carotin-Zubereitungen ist ersichtlich, dass der Zusatz von Acetylcystein nicht nur Beta-Carotin, sondern auch den in der Zubereitung enthaltenen Anteil an Konservierungsmitteln (das "Konservierungssystem") vor Abbau schützt, also die Stabilität der Zubereitung erhöht.

Zusammenfassend kann eine gemäß der Erfindung herstellbare Emulsion wie folgt beschrieben werden:
Eine, in der Veterinärmedizin verwendbare Beta-Carotin enthaltende wässerige Emulsion zur parenteralen Verabreichung aus einem sterilen Mehrdosisbehältnis enthält als die Emulsion stabilisierenden sowie Konservierungsmittel und Beta-Carotin vor Abbau schützenden Stoff Acetylcystein in Mengen zwischen 1 und 8 Gew.%. Zusätzlich enthält die Beta-Carotin enthaltende Emulsion wenigstens ein Antioxidans und gegebenenfalls wenigstens ein Konservierungsmittel. Als Antioxidans können Ascorbylpalmitat oder Alpha-Tocopherol zugesetzt werden. Weiters kann die Emulsion Lösungsvermittler, beispielsweise Isopropylmyristat oder Solutol HS 15, enthalten.

## Patentansprüche

1. Verfahren zum Herstellen einer zur parenteralen Verabreichung bestimmten Emulsion von Beta-Carotin in einem wässerigen Medium, die, bezogen auf die Emulsion in Mengen zwischen 1 und 8 Gew.% Acetylcystein, vorzugsweise 2 bis 5 Gew.%, besonders bevorzugt 3 Gew.%, Acetylcystein, einen Lösungsvermittler, insbesondere Isopropylmyristat, und wenigstens ein Antioxidans enthält, **gekennzeichnet durch** folgende Verfahrensschritte:
a) Erwärmen des nicht-ionischen Lösungsvermittlers, um den nicht-ionischen Lösungsvermittler zu schmelzen, und Zusetzen von Isopropylmyristat zu dem geschmolzenen Lösungsvermittler,
b) Einrühren von Beta-Carotin in die in Schritt a) erhaltene Lösung,
c) Eintragen der in Schritt b) erhaltenen Beta-Carotin enthaltende Mischung in Wasser unter Rühren,
d) Rühren des in Schritt c) erhaltenen Gemisches, bis eine dunkelrote, klare Emulsion vorliegt,
e) Lösen von wenigstens einem Antioxidans, insbesonders in einem organischen Lösungsmittel, das gleichzeitig als Konservierungsmittel dient, wie Benzylalkohol,
f) Einrühren der in Schritt e) erhaltenen Lösung des Antioxidans in die, in Schritt d) erhaltene Beta-Carotin enthaltende Emulsion und
g) Zugeben von Acetylcystein in die in Schritt f) erhaltene Emulsion.

2. Verfahren nach Anspruch 1, **dadurch gekennzeichnet, dass** der nicht-ionische Lösungsvermittler auf eine Temperatur zwischen 135° Celsius und 140° Celsius erhitzt wird, bevor Isopropylmyristat zugesetzt wird.

3. Verfahren nach Anspruch 2, **dadurch gekennzeichnet, dass** dem geschmolzenen Lösungsvermittler Isopropylmyristat bei einer Temperatur zwischen 125 und 140° Celsius, insbesondere bei 130° Celsius, zugesetzt wird.

4. Verfahren nach Anspruch 2 oder 3, **dadurch gekennzeichnet, dass** die im Schritt b) erhaltene Mischung enthaltend Beta-Carotin, den geschmolzenen nicht-ionischen Lösungsvermittler und Isopropylmyristat in erwärmtes Wasser eingetragen und gerührt wird, bis eine dunkelrote klare Emulsion vorliegt.

5. Verfahren nach einem der Ansprüche 1 bis 4, **dadurch gekennzeichnet, dass** in Schritt b) bei einer Temperatur von 90° Celsius gerührt wird.

6. Verfahren nach einem der Ansprüche 1 bis 5, **dadurch gekennzeichnet, dass** der in Schritt d) erhaltenen Beta-Carotin enthaltenden Emulsion eine Lösung wenigstens eines Antioxidans bei einer Temperatur von etwa 50° zugesetzt wird.

7. Verfahren nach Anspruch 6, **dadurch gekennzeichnet, dass** der mit Antioxidans versetzten Emulsion bei einer Temperatur von 30° Celsius Acetylcystein, insbesondere in Wasser gelöstes Acetylcystein, zugemischt wird.

8. Verfahren nach einem der Ansprüche 1 bis 7, **dadurch gekennzeichnet, dass** die Verfahrensschritte, mit Ausnahme des ersten Verfahrensschrittes a), unter Stickstoffatmosphäre ausgeführt werden.

9. Verfahren nach einem der Ansprüche 1 bis 8, **dadurch gekennzeichnet, dass** in Schritt a) als nicht-ionische Lösungsvermittler Hydroxyethyl-12-hydroxyoktadekanoat und Macrogol-15-hydroxystearat (Solutol HS 15) eingesetzt werden.

10. Verfahren nach einem der Ansprüche 1 bis 9, **dadurch gekennzeichnet, dass** in Schritt e) als Antioxidans Ascorbylpalmitat und/oder Alpha-Tocopherol und als Konservierungsmittel Benzylalkohol eingesetzt werden.

## Claims

1. Method for producing an emulsion of beta-carotene in an aqueous medium, the emulsion being intended for parenteral administration and containing acetylcysteine in quantities between 1 and 8 wt% relative to the emulsion, preferably 2 to 5 wt%, particularly preferably 3 wt% acetylcysteine, a solubilizer, in particular isopropyl myristate, and at least one antioxidant, **characterized by** the following method steps:
a) heating the nonionic solubilizer in order to melt the nonionic solubilizer, and adding isopropyl myristate to the molten solubilizer,
b) stirring beta-carotene into the solution obtained in step a),
c) introducing the beta-carotene-containing mixture obtained in step b) into water while stirring,
d) stirring the mixture obtained in step c) until a dark red, clear emulsion is achieved,
e) dissolving at least one antioxidant, in particular in an organic solvent which simultaneously serves as a preservative, such as benzyl alcohol,
f) stirring the solution of the antioxidant obtained in step e) into the beta-carotene-containing emulsion obtained in step d), and
g) adding acetylcysteine to the emulsion obtained in step f) .

2. Method according to claim 1, **characterized in that** the nonionic solubilizer is heated to a temperature between 135° Celsius and 140° Celsius before isopropyl myristate is added.

3. Method according to claim 2, **characterized in that** isopropyl myristate is added to the molten solubilizer at a temperature between 125 and 140° Celsius, in particular at 130° Celsius.

4. Method according to claim 2 or 3, **characterized in that** the mixture obtained in step b), which contains beta carotene, the molten nonionic solubilizer and isopropyl myristate, is introduced into heated water and stirred until a dark red, clear emulsion is achieved.

5. Method according to any one of claims 1 to 4, **characterized in that**, in step b), stirring takes place at a temperature of 90° Celsius.

6. Method according to any one of claims 1 to 5, **characterized in that** a solution of at least one antioxidant is added to the beta-carotene-containing emulsion obtained in step d) at a temperature of approximately 50°.

7. Method according to claim 6, **characterized in that** acetylcysteine, in particular acetylcysteine dissolved in water, is mixed into the emulsion, to which antioxidant has been added, at a temperature of 30° Celsius.

8. Method according to any one of claims 1 to 7, **characterized in that** the method steps, with the exception of the first method step a), are carried out under a nitrogen atmosphere.

9. Method according to any one of claims 1 to 8, **characterized in that**, in step a), hydroxyethyl-12-hydroxyoctadecanoate and macrogol-15-hydroxystearate (Solutol HS 15) are used as nonionic solubilizers.

10. Method according to any one of claims 1 to 9, **characterized in that**, in step e), ascorbyl palmitate and/or alpha-tocopherol are used as antioxidants and benzyl alcohol is used as preservative.

## Revendications

1. Procédé pour la préparation d'une émulsion de bêta-carotène dans un véhicule aqueux pour une administration parentérale, qui contient, par rapport à l'émulsion, entre 1 et 8 % en poids d'acétylcystéine, de préférence entre 2 et 5 % en poids, en particulier 3 % en poids d'acétylcystéine, un agent de solubilisation, en particulier du myristate d'isopropyle, et au moins un antioxydant, **caractérisé en ce qu'**il comprend les étapes de précédé suivantes :
a) chauffage de l'agent de solubilisation non ionique pour le faire fondre et ajout de myristate d'isopropyle à l'agent de solubilisation fondu,
b) ajout et agitation de bêta-carotène dans la solution obtenue dans l'étape a),
c) incorporation du mélange contenant du bêta-carotène obtenu dans l'étape b) dans de l'eau en remuant,
d) agitation du mélange obtenue dans l'étape c) jusqu'à obtention d'une émulsion limpide rouge foncé,
e) dissolution d'au moins un antioxydant, en particulier dans un solvant organique qui sert en même temps de conservateur, comme de l'alcool benzylique,
f) incorporation et agitation de la solution d'antioxydant obtenue dans l'étape e) dans l'émulsion contenant du bêta-carotène obtenue dans l'étape d) et
g) ajout d'acétylcystéine dans l'émulsion obtenue dans l'étape f).

2. Procédé selon la revendication 1, **caractérisé en ce que** l'agent de solubilisation non ionique est chauffé à une température comprise entre 135° Celsius et 140° Celsius avant l'ajout du myristate d'isopropyle.

3. Procédé selon la revendication 2, **caractérisé en ce que** du myristate d'isopropyle est ajouté à l'agent de solubilisation fondu à une température comprise entre 125° Celsius et 140° Celsius, en particulier à 130° Celsius.

4. Procédé selon la revendication 2 ou 3, **caractérisé en ce que** le mélange obtenu dans l'étape b) contenant du bêta-carotène, l'agent de solubilisation non ionique fondu et du myristate d'isopropyle est incorporé dans de l'eau chauffée et agité jusqu'à obtention d'une émulsion limpide rouge foncé.

5. Procédé selon l'une des revendications 1 à 4, **caractérisé en ce que** dans l'étape b), l'agitation a lieu à une température de 90° Celsius.

6. Procédé selon l'une des revendications 1 à 5, **caractérisé en ce qu'**une solution contenant au moins un antioxydant est ajoutée à l'émulsion contenant du bêta-carotène obtenue dans l'étape d) à une température d'environ 50°.

7. Procédé selon la revendication 6, **caractérisé en ce que** l'émulsion additionnée d'antioxydant est mélangée à de l'acétylcystéine, en particulier de l'acétylcystéine dissoute dans l'eau, à une température de 30° Celsius.

8. Procédé selon l'une des revendications 1 à 7, **caractérisé en ce que** les étapes de procédé, à l'exception de la première étape a), sont exécutées sous atmosphère d'azote.

9. Procédé selon l'une des revendications 1 à 8, **caractérisé en ce que** l'agent de solubilisation non ionique utilisé dans l'étape a) est du 12-hydroxyoctadécanoate d'hydroxyéthyle et du 15-hydroxystéarate de macrogol (Solutol HS 15).

10. Procédé selon l'une des revendications 1 à 9, **caractérisé en ce que** l'antioxydant utilisé dans l'étape e) est du palmitate d'ascorbyle et/ou de l'alpha-tocophérol et le conservateur est de l'alcool benzylique.
